# EUROPEAN PATENT APPLICATION

(11) **EP 3 984 995 A1**
(43) Date of publication of application: **20.04.2022**
(21) Application number: 20306217.9
(22) Date of filing: 15.10.2020
(51) Int. Cl.: C07D 219/02

(54) **ACRIDINIUM-BASED PHOTOREDOX CATALYSTS, SYNTHESIS AND USE THEREOF IN OXIDATIVE CLEAVAGE OF C-O BONDS**

(71) Applicant: Université de Rennes 1, 35000 Rennes (FR); Centre national de la recherche scientifique, 75016 Paris (FR); École Nationale Supérieure De Chimie, 35000 Rennes (FR); Institut National des Sciences Appliquées de Rennes, 35700 Rennes (FR)
(72) Inventor: SOULÉ, Jean-François, 35000 Rennes (FR)
(74) Representative: Novagraaf Technologies

(57) **Abstract**

The present invention belongs to the field of catalytic chemistry, and more specifically to catalysed oxidation of lignin. It also relates to synthesis of catalyst compounds.

The present invention relates to new acridinium-based photoredox catalyst compounds and their use thereof in a chemical reaction, preferably in depolymerisation of lignin models and ultimately lignin. The invention also relates to the method of synthesis of the new acridinium-based photoredox catalyst compounds according to the invention.

## Description

### Technical field

The present invention belongs to the field of catalytic chemistry, and more specifically to catalysed oxidation of lignin. It also relates to synthesis of catalyst compounds.

The present invention relates to new acridinium-based photoredox catalyst compounds and their use thereof in a chemical reaction, preferably in depolymerisation of lignin models and ultimately lignin. The invention also relates to the method of synthesis of the new acridinium-based photoredox catalyst compounds according to the invention.

In the description below, references between **[ ]** refer to the list of references at the end of the examples.

### Technical background

Lignin is a highly oxygenated polyphenolic biopolymer which constitutes an attractive renewable feedstock for the production of bio-sourced aromatic building blocks that are traditionally obtained from fossil-based feedstocks **[1].** The current chemical techniques to depolymerize lignin do not enable the production of highly selective single aromatic building blocks because its structure contains different linkages and monomers that often leads to a myriad of complex mixtures of products **[2].** One way to solve this matter is to develop new catalysts able to achieve C-O bond cleavage with a high degree of selectivity.

Besides the classical strategies for lignin depolymerization (e.g., pyrolysis, hydrolysis, enzymolysis, hydrogenolysis, and oxidation) photoredox catalysis has become a powerful tool for the selective C-O bond cleavage of lignin β-O-4 model compounds **[3-8].** However, they required the pre-oxidation of the benzylic alcohols. In these cases, the C(sp³)-O bond is braked affording acetophenone and phenol derivatives.

Developing new photocatalysts enabling alternative C-O bond cleavages of lignin β-O-4 model compounds to afford 1,2-diol derivatives represents a major socioeconomic stake for chemical companies and an important challenge for academic research group because bio-polyols derived from Lignin would be suitable to replace or partly replace petroleum-based polyols in polyurethane foam synthesis **[9].** So far, only one catalytic protocol is reported using expensive cobalt deuteroporphyrin complex as a biomimetic catalyst and 5 equivalents of Oxone^{®} as oxidant **[10].**

Since the pioneering work in 2006 by Fukuzumi and co-workers on the use of 9-phenyl-10-methylacridium as an active organic photocatalyst (OPC) for solvent-free selective photocatalytic oxidation of benzyl alcohol to benzaldehyde under visible light irradiation, several acridinium-based catalysts have been designed to also oxidize secondary benzylic alcohols **[11-12].**

To the best of our knowledge, there is no report for the catalytic oxidation of lignin with acridinium-based photoredox catalyst. Focusing to develop new methods for catalytic biomass conversion, we report herein a novel and efficient acridinium-based photoredox catalyst for the oxidative C-O bond cleavage of lignin model compounds without peroxidation step, therefore improving previously known catalysts and associated reactions.

### Detailed description of the invention

Applicant has developed new catalyst compounds that solves all of the problems listed above.

The present invention deals with acridinium-based photoredox catalyst compounds, their synthetic methods and their applications, such as depolymerisation of lignin.

The catalyst of the invention presents the advantage to be easily synthesised, in mild conditions and present a photoredox catalytic activity for the oxidative C-O bond cleavage of lignin model compounds without peroxidation step activity that is superior to known catalysts, in particular superior to the most commonly used, such as the Fukuzumi catalyst.

The invention therefore relates to a compound of formula I: 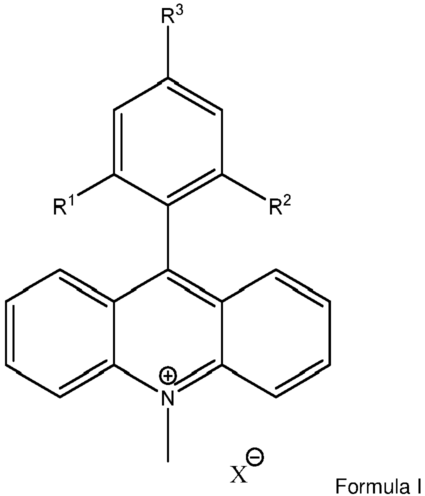 wherein,
- the R¹ and R² groups independently represent H, a halo group or a phenyl group,
- the R³ group represents H, a halo group, a C1 to C4 linear or branched alkyl chain, a - COOR group, a -COR group or a phenyl group, in which R represents a C1 to C4 linear or branched alkyl chain, and
- X⁻ represents an anion.

It is meant by a "halo group", a group derived from a halogen. Preferably, the halogen may be chosen in the group comprising fluorine, chlorine, bromine or iodine, preferably bromine or iodine. The halo group may thus be chosen in the group comprising fluoro, chloro, bromo or iodo group, preferably bromo or iodo group.

Advantageously, the R¹ and R² groups independently represents H, a halo group or a phenyl group. Preferably, the R¹ and R² groups may independently represent H, a bromo, a iodo or a phenyl group.

Advantageously, the R³ group represents H, a halo group, a C1 to C4 linear or branched alkyl chain, a -COOR group, a -COR group or a phenyl group, in which R represents a C1 to C4 linear or branched alkyl chain. Preferably, the R³ group may represent H, a bromo group, -COOEt, -COMe or Me.

Advantageously, the compound according to the invention may be a compound of formula I wherein at least one group amongst R¹, R² and R³ is not H.

Advantageously, the compound according to the invention may be a compound of formula I wherein R¹ may be H, a bromo, a iodo or a phenyl group, R² may be H and R³ may be H, a bromo group, -COOEt, -COMe or Me, and R¹ and R³ may not be H simultaneously.

Advantageously, the compound according to the invention may be chosen from the compounds of formula I wherein R² and R³ are H, of formula II. The compound may be of formula II: 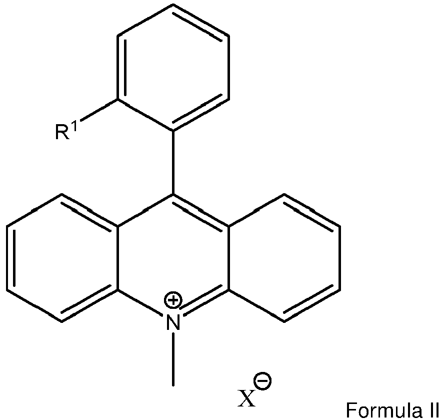 wherein,
- the R¹ group represents a halo group or a phenyl group, and
- X⁻ represents an anion. Preferably, R¹ may be a bromo or a iodo group.

Advantageously, the compound according to the invention may be chosen from the compounds of formula I wherein R¹ and R² are H, of formula III. The compound may be of formula III: 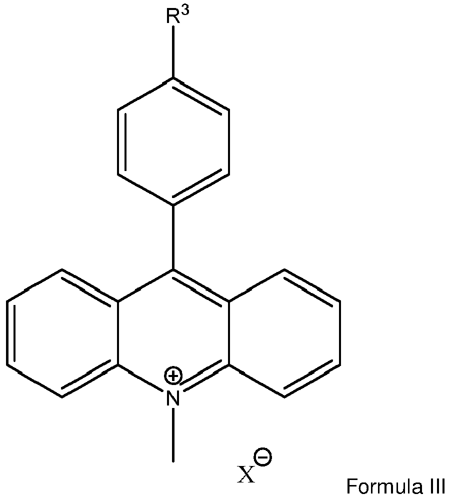 wherein,
- the R³ group represents a halo group, a C1 to C4 linear or branched alkyl chain, a - COOR group a -COR group or a phenyl group, in which R is a C1 to C4 linear or branched alkyl chain, and
- X⁻ represents an anion. Preferably, R³ may be a bromo group, -COOEt, -COMe or Me.

Advantageously, the compound according to the invention may be chosen in the group of compounds of formula I, II or III wherein the anion X⁻ is chosen in the group comprising tetraborate (BF₄⁻), hexafluorophosphate (PF₆⁻), chloride (Cl⁻) and perchlorate (ClO₄⁻), preferably BF₄⁻.

The invention further relates to a use of a compound according to the invention as a catalyst, preferably in a C-O bond cleavage reaction, and more preferably a photocatalytic C-O bond cleavage.

Advantageously, the use according to the invention may be the use of a compound according to the invention in a reaction of lignin depolymerisation, preferably in oxidative depolymerisation of lignin.

Advantageously, the invention may relate to a method of C-O bond cleavage, preferably a method of photocatalytic C-O bond cleavage comprising a step of using a compound according to the invention.

Advantageously, the invention may relate to a reaction of lignin depolymerisation, preferably an oxidative depolymerisation of lignin, comprising a step of using a compound according to the invention.

Advantageously, in the method or use according to the invention, the C-O bond cleavage reaction or the reaction of lignin depolymerisation may be done, by irradiation of lignin or a lignin model, in an organic solvent, in the presence of a compound according to the invention. The organic solvent may be chosen in the group comprising methanol (MeOH), dimethylsulfoxide (DMSO), acetonitrile (ACN), dichloromethane (DCM) and mixtures thereof. The C-O bond cleavage reaction or the reaction of lignin depolymerisation may be done by photoreacting lignin or a lignin model in the presence of a compound according to the invention by means of irradiation at a wavelength comprised in the range of visible light (i.e. from 400 to 800 nm), preferably from 440 to 460 nm. The source of light may be a LED, preferably a blue light LED. Irradiation may last from 6 hours to 20 hours, preferably 12 hours. The C-O bond cleavage reaction or the reaction of lignin depolymerisation may be done at a temperature comprised in a range from 10 to 45 °C, preferably 30 ºC.

The invention also relates to a synthetic method of a compound of formula I comprising a step of photoreacting, in an organic solvent, a 10-methylacridinium salt and a diazonium salt of formula I': 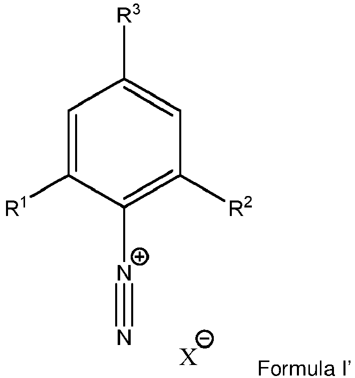 wherein,
- the R¹ and R² groups independently represent H, a halo group or a phenyl group,
- the R³ group represents H, a halo group, a C1 to C4 linear or branched alkyl chain, a - COOR group, a -COR group or a phenyl group, in which R represents a C1 to C4 linear or branched alkyl chain, and
- X⁻ represents an anion.

Advantageously, the R¹ and R² groups independently represents H, a halo group or a phenyl group. Preferably, the R¹ and R² groups may independently represent H, a bromo, a iodo or a phenyl group.

Advantageously, the R³ group represents H, a C1 to C4 linear or branched alkyl chain, a halo group, a -COOR group, a -COR group or a phenyl group, in which R represents a C1 to C4 linear or branched alkyl chain. Preferably, the R³ group may H, a bromo group, -COOEt, -COMe or Me.

Advantageously, the synthetic method according to the invention may be a synthetic method of a compound of formula I comprising a step of photoreacting, in an organic solvent, a 10-methylacridinium salt and a diazonium salt of formula I', wherein R¹ may be H, a bromo, a iodo or a phenyl group, R² may be H and R³ may be H, a bromo group, -COOEt, -COMe or Me, and R¹ and R³ may not be H simultaneously.

Advantageously, the synthetic method according to the invention may be a synthetic method of a compound of formula I comprising a step of photoreacting, in an organic solvent, a 10-methylacridinium salt and a diazonium salt of formula I', wherein at least one group amongst R¹, R² and R³ is not H.

Advantageously, the invention may also relate to a synthetic method of a compound of formula II comprising a step of photoreacting, in an organic solvent, a 10-methylacridinium salt and a diazonium salt of formula II': 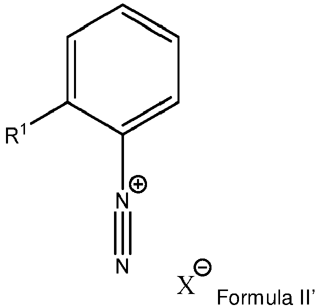 wherein
- the R¹ group represents a halo group or a phenyl group, and
- X⁻ represents an anion. Preferably, R¹ may be a bromo or a iodo group.

Advantageously, the invention may also relate to a synthetic method of a compound of formula III comprising a step of photoreacting, in an organic solvent, a 10-methylacridinium salt and a diazonium salt of formula III': 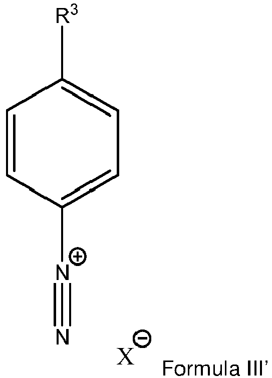 wherein,
- the R³ group represents a halo group, a C1 to C4 linear or branched alkyl chain, a COOR group, a COR group or a phenyl group, in which R is a C1 to C4 linear or branched alkyl chain, and
- X⁻ represents an anion. Preferably, R³ may be a bromo group, -COOEt, -COMe or Me.

Advantageously, in the synthetic method according to the invention, the compound may be chosen from the compounds of formula I', II' or III' wherein the anion X⁻ is chosen in the group comprising tetraborate (BF₄⁻), hexafluorophosphate (PF₆⁻), chloride (Cl⁻) and perchlorate (ClO₄⁻), preferably BF₄⁻.

Advantageously, in the synthetic method according to the invention, the step of photoreacting a 10-methylacridinium salt and a diazonium salt of formula I', II' or III' is carried out in an organic solvent. The organic solvent may be chosen in the group comprising methanol (MeOH), dimethylsulfoxide (DMSO), acetonitrile (ACN), dichloromethane (DCM) and mixtures thereof. The concentration of 10-methyl-9-phenylacridinium salt may be from 0.12 to 1.2 mol/L. The concentration of diazonium salt may be from 0.1 to 1 mol/L.

Advantageously, the 10-methylacridinium salt may be chosen in the group comprising 10-methylacridinium tetraborate (BF₄⁻), 10-methylacridinium hexafluorophosphate (PF₆⁻), 10-methylacridinium chloride (Cl⁻) and 10-methylacridinium perchlorate (ClO₄⁻), preferably 10-methylacridinium tetraborate.

Advantageously, in the synthetic method according to the invention, the step of photoreacting a 10-methylacridinium salt and a diazonium salt of formula I', II' or III' may be done by means of irradiation at a wavelength comprised in the range of visible light (i.e. from 400 to 800 nm), preferably from 440 to 460 nm. The source of light may be a LED, preferably a blue light LED. Irradiation may last from 30 minutes to 2 hours, preferably 1 hour.

Advantageously, in the synthetic method according to the invention, the step of photoreacting a 10-methylacridinium salt and a diazonium salt of formula I', II' or III' may be done at a temperature comprised in a range from 10 to 45 °C, preferably 30 ºC.

Advantageously, the synthetic method may further comprise a step of quenching the reaction by addition of an organic solvent (preferably CH₂Cl₂). The quenching of the reaction may be followed by a step of washing the crude mixture with water and removing the solvent under reduced pressure. The obtained catalyst compound may be further purified by flash column chromatography on silica gel.

### EXAMPLES

### Example 1: Preparation of OPC1-8 according to the invention

All reactions were carried out under argon atmosphere with standard Schlenk techniques. All reagents were obtained from commercial sources and used as supplied. Technical grade petroleum ether (40-60) and ethyl acetate were used for column chromatography. 1 H NMR spectra were recorded on Bruker GPX (300, 400 or 500 MHz) spectrometer. Chemical shifts (δ) were reported in parts per million relative to residual chloroform (7.28 ppm for 1 H; 77.23 ppm for 13C), constants were reported in Hertz. ¹H NMR assignment abbreviations were the following: singlet (s), doublet (d), triplet (t), quartet (q), doublet of doublets (dd), doublet of triplets (dt), and multiplet (m). 13C NMR spectra were recorded at 100 MHz on the same spectrometer and reported in ppm. GC-analyses were performed with a GC-2010 (Shimadzu) instrument equipped with a 30 m capillary column (Agilent J&W DB-FFAP, 15m x 0,250 mm x 0,25 µm), which was used with helium as the vector gas. The following GC conditions were used: initial temperature 80 ºC for 5 minutes, then rate 20 ºC/min until 280 ºC and 280 ºC for 28 minutes. HRMS were recorded on a Waters Q-Tof 2 mass spectrometer at the corresponding facilities of the CRMPO, Centre Régional de Mesures Physiques de l'Ouest, Université de Rennes 1.

### General procedure for the preparation of acridinium-based photoredox catalysts (OPC compounds):

To a 25 mL Schlenk tube was added 10-methylacridinium tetrafluoroborate (168 mg, 0.6 mmol, 1.2 equiv), and diazonium salts (0.5 mmol, 1 equiv) under air. The air was evacuated and backfilled with argon (3 times). Freshly distilled DMSO (1 mL) and MeOH (1 mL) was added. The Schlenk tube was then sealed under argon with a Teflon lined cap. The Schlenk was positioned on a stir plate approximately 2 - 3 cm from two ABI PAR38 (24W) LED lamp supplying blue light (λ = 440- 460 nm). Fans were used to maintain the temperature around 30 ºC. After 1 hour irradiation, the reaction was quenched by addition of 20 ml CH₂Cl₂ and the crude mixture was washed with 3mL cold water 3 times, then the solvent was removed under reduced pressure and purified by flash column chromatography on silica gel/Al₂O₃ to give pure product. Results are presented in table 1 below.

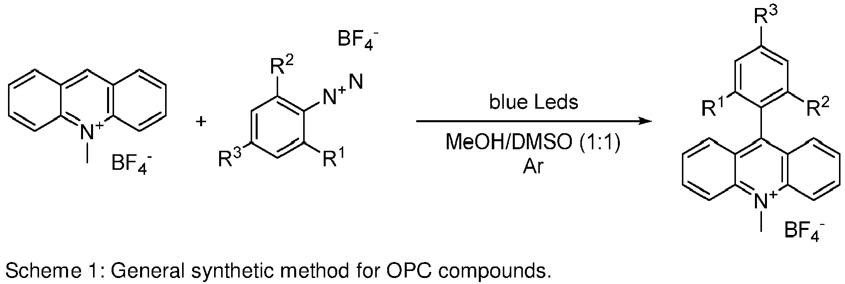

**Table 1: OPC compounds yields (%) based on diazonium salt.**

| OPC compound | 10-methylacridinium tetrafluoroborate | diazonium salt | R¹ = | Yields (%) based on diazonium salt |
|---|---|---|---|---|
| | | | R² = | |
| | | | R³ = | |
| OPC1 | 0.6 mmol | 0.5 mmol | R¹ = H | 80 |
| | | | R² = H | |
| | | | R³ = H | |
| OPC2 | 0.6 mmol | 0.5 mmol | R¹ = H | 68 |
| | | | R² = H | |
| | | | R³ = Br | |
| OPC3 | 0.6 mmol | 0.5 mmol | R¹ = H | 65 |
| | | | R² = H | |
| | | | R³ = COOEt | |
| OPC4 | 0.6 mmol | 0.5 mmol | R¹ = H | 71 |
| | | | R² = H | |
| | | | R³ = COMe | |
| OPC5 | 0.6 mmol | 0.5 mmol | R¹ = Br | 58 |
| | | | R² = H | |
| | | | R³ = H | |
| OPC6 | 0.6 mmol | 0.5 mmol | R¹ = I | 57 |
| | | | R² = H | |
| | | | R³ = H | |
| OPC7 | 0.6 mmol | 0.5 mmol | R¹ = Ph | 79 |
| | | | R² = H | |
| | | | R³ = H | |
| OPC8 | 0.6 mmol | 0.5 mmol | R¹ = Br | 56 |
| | | | R² = H | |
| | | | R³ = Me | |

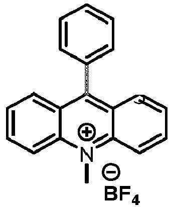

### 10-Methyl-9-phenylacridinium tetrafluoroborate (OPC1):

Following the general procedure using phenyl diazonium tetrafluoroborate (96 mg, 0.5 mmol), the residue was purified by flash chromatography on silica gel to afford the desired compound **OPC1** (143 mg, 80%) as a yellow solid. ¹H NMR (400 MHz, Acetone-*d*₆) δ 8.94 (d, *J* = 9.3 Hz, 2H), 8.64 - 8.47 (m, 2H), 8.10 (d, *J* = 8.5 Hz, 2H), 8.05 - 7.96 (m, 2H), 7.82 (dd, *J* = 4.9, 1.7 Hz, 3H), 7.66 (dd, *J* = 6.4, 3.0 Hz, 2H), 5.15 (s, 3H). ¹¹B NMR (128 MHz, Acetone-*d*₆) δ -0.90. ¹³C NMR (101 MHz, Acetone-*d*₆) δ 162.4, 142.6, 139.5, 134.2, 131.1, 130.9, 130.7, 129.7, 128.7, 127.0, 119.6, 39.5.¹⁹F NMR (376 MHz, Acetone-*d*₆) δ -151.86.

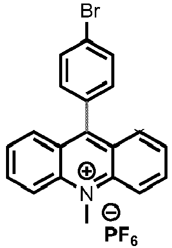

### 9-(4-Bromophenyl)-10-methylacridinium tetrafluoroborate (OPC2):

Following the general procedure using 4-bromobenzenediazonium tetrafluoroborate (135 mg, 0.5 mmol), the residue was purified by flash chromatography on silica gel to afford the desired compound **OPC2** (148 mg, 68%) as a yellow solid. ¹H NMR (400 MHz, Acetone-*d*₆) δ 8.95 (d, *J* = 9.2 Hz, 2H), 8.59 - 8.47 (m, 2H), 8.13 (d, *J* = 8.5 Hz, 2H), 8.07 - 7.93 (m, 4H), 7.63 (d, *J* = 8.2 Hz, 2H), 5.14 (s, 3H). ¹¹B NMR (128 MHz, Acetone-*d*₆) δ -0.94. ¹³C NMR (101 MHz, Acetone-*d*₆) δ 160.9, 142.6, 139.6, 133.4, 132.9, 132.7, 130.7, 128.8, 126.8, 125.0, 119.7, 39.6. ¹⁹F NMR (376 MHz, Acetone-*d*₆) δ -151.80. HRMS (ESI) (*m*/*z*): [M]⁺ calcd. For C₂₀H₁₅N⁷⁹Br: 348.0382, found: 348.0380.

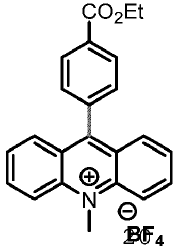

### 9-(4-(Ethoxycarbonyl)phenyl)-10-methylacridinium tetrafluoroborate (OPC3):

Following the general procedure using 4-(ethoxycarbonyl)benzenediazonium tetrafluoroborate (132 mg, 0.5 mmol), the residue was purified by flash chromatography on silica gel to afford the desired compound **OPC3** (139 mg, 65%) as a yellow solid. ¹H NMR (400 MHz, Methylene Chloride-*d*₂) δ 8.66 (d, *J* = 9.2 Hz, 2H), 8.47 - 8.35 (m, 4H), 8.02 - 7.95 (m, 2H), 7.90 - 7.80 (m, 2H), 7.61 (d, *J* = 8.3 Hz, 2H), 4.99 (s, 3H), 4.48 (q, *J* = 7.1 Hz, 2H), 1.47 (t, *J* = 7.1 Hz, 3H). ¹¹B NMR (128 MHz, Methylene Chloride-*d*₂) δ -1.20. ¹³C NMR (101 MHz, Methylene Chloride-*d*₂) δ 165.3, 160.5, 141.4, 139.2, 137.0, 132.6, 129.9, 129.9, 129.8, 128.0, 125.7, 118.3, 61.6, 39.0, 14.0. ¹⁹F NMR (376 MHz, Methylene Chloride-*d*₂) δ -153.04. HRMS (ESI) (*m*/*z*): [M]+ calcd. For C₂₃H₂₀NO₂: 342.1488, found: 342.1492.

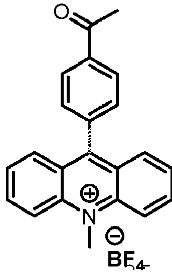

### 9-(4-Acetylphenyl)-10-methylacridinium tetrafluoroborate (OPC4):

Following the general procedure using 4-acetylbenzenediazonium tetrafluoroborate (117 mg, 0.5 mmol), the residue was purified by flash chromatography on silica gel to afford the desired compound **OPC4** (106 mg, 71%) as a green solid. ¹H NMR (400 MHz, Methylene Chloride-*d*₂) δ 8.66 (d, *J* = 9.2 Hz, 2H), 8.47 - 8.36 (m, 2H), 8.29 (d, *J* = 8.2 Hz, 2H), 7.99 (d, *J* = 9.4 Hz, 2H), 7.93 - 7.79 (m, 2H), 7.64 (d, *J* = 8.3 Hz, 2H), 4.98 (s, 3H), 2.75 (s, 3H). ¹¹B NMR (128 MHz, Methylene Chloride-*d*₂) δ -1.19. ¹³C NMR (101 MHz, Methylene Chloride-*d*₂) δ 197.5, 160.9, 142.0, 139.8, 139.1, 137.7, 130.7, 130.43, 129.1, 128.6, 126.2, 118.9, 39.5, 27.1. ¹⁹F NMR (376 MHz, Methylene Chloride-*d*₂) δ -152.81. HRMS (ESI) (*m*/*z*): [M]+ calcd. For C₂₂H₁₈NO: 312.1383, found: 312.1380.

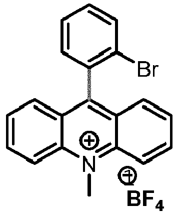

### 9-(2-Bromophenyl)-10-methylacridinium tetrafluoroborate (OPC5):

Following the general procedure using 2-bromobenzenediazonium tetrafluoroborate (135 mg, 0.5 mmol), the residue was purified by flash chromatography on silica gel to afford the desired compound **OPC5** (126 mg, 58%) as a yellow solid. ¹H NMR (400 MHz, Methylene Chloride-*d*₂) δ 8.68 (d, *J* = 9.2 Hz, 2H), 8.52 - 8.31 (m, 2H), 8.14 - 7.79 (m, 5H), 7.76 - 7.62 (m, 2H), 7.48 -7.36 (m, 1H), 5.00 (s, 3H). ¹¹B NMR (128 MHz, Methylene Chloride-*d*₂) δ -1.13. ¹³C NMR (101 MHz, Methylene Chloride-*d*₂) δ 159.9, 141.5, 139.3, 133.9, 133.4, 132.1, 131.1, 129.6, 128.3, 128.1, 125.7, 122.2, 118.4, 39.0. ¹⁹F NMR (376 MHz, Methylene Chloride-*d*₂) δ -152.96. HRMS (ESI) (*m*/*z*): [M]+ calcd. For C₂₀H₁₅N⁷⁹Br: 348.0382, found: 348.0383.

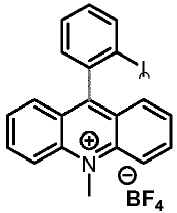

### 9-(2-lodophenyl)-10-methylacridin-10-ium tetrafluoroborate (OPC6):

Following the general procedure using 2-iodobenzenediazonium tetrafluoroborate (159 mg, 0.5 mmol), the residue was purified by flash chromatography on silica gel to afford the desired compound **OPC6** (138 mg, 57%) as a yellow solid. ¹H NMR (400 MHz, Methylene Chloride-*d*₂) δ 8.67 (d, *J* = 9.3 Hz, 2H), 8.52 - 8.35 (m, 2H), 8.20 (d, *J* = 8.6 Hz, 1H), 7.86 (d, *J* = 4.1 Hz, 4H), 7.77 - 7.68 (m, 1H), 7.52 - 7.33 (m, 2H), 4.98 (s, 3H). ¹¹B NMR (128 MHz, Methylene Chloride-*d*₂) δ -1.15. ¹³C NMR (101 MHz, Methylene Chloride-*d*₂) δ 162.3, 141.6, 139.8, 139.3, 138.0, 131.8, 130.4, 129.7, 128.7, 128.2, 125.6, 118.4, 96.6, 39.0. ¹⁹F NMR (376 MHz, Methylene Chloride-*d*₂) δ - 152.88.

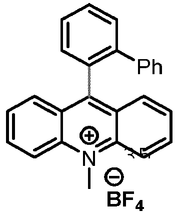

### 9-([1,1'-Biphenyl]-2-yl)-10-methylacridinium tetrafluoroborate (OPC7):

Following the general procedure using 2-phenylbenzenediazonium tetrafluoroborate (134 mg, 0.5 mmol), the residue was purified by flash chromatography on silica gel to afford the desired compound **OPC7** (171 mg, 79%) as a yellow solid. ¹H NMR (400 MHz, DMSO-*d*₆) δ 8.78 (d, *J* = 9.2 Hz, 2H), 8.45 - 8.34 (m, 2H), 8.00 (d, *J* = 8.2 Hz, 2H), 7.94 - 7.84 (m, 3H), 7.76 (t, *J* = 7.1 Hz, 2H), 7.49 (d, *J* = 7.3 Hz, 1H), 6.98 (s, 5H), 4.88 (s, 3H). ¹¹B NMR (128 MHz, DMSO-*d*₆) δ -1.27. ¹⁹F NMR (376 MHz, DMSO-*d*₆) δ - 148.35. ¹³C NMR (101 MHz, DMSO-*d*₆) δ 160.5, 141.4, 140.8, 139.3, 138.4, 131.6, 130.9, 130.8, 130.7, 129.6, 128.3, 128.1, 127.8, 127.4, 125.6, 119.3, 39.0.

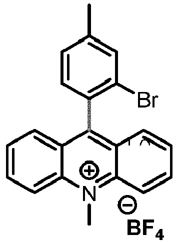

### 9-(2-Bromo-4-methylphenyl)-10-methylacridinium tetrafluoroborate (OPC 8):

Following the general procedure using 2-bromo-4-methylbenzenediazonium tetrafluoroborate (142 mg, 0.5 mmol), the residue was purified by flash chromatography on silica gel to afford the desired compound **OPC8** (126 mg, 56%) as a yellow solid. ¹H NMR (400 MHz, Methylene Chloride-*d*₂) δ 8.66 (d, *J* = 9.2 Hz, 2H), 8.50 - 8.35 (m, 2H), 7.99 - 7.75 (m, 5H), 7.51 (d, *J* = 7.5 Hz, 1H), 7.29 (d, *J* = 7.8 Hz, 1H), 4.98 (s, 3H), 2.57 (s, 3H). ¹¹B NMR (128 MHz, Methylene Chloride-*d*₂) δ -1.13. ¹³C NMR (101 MHz, Methylene Chloride-*d*₂) δ 160.4, 143.1, 141.5, 139.3, 133.8, 130.8, 130.7, 129.8, 128.8, 128.2, 125.9, 121.8, 118.4, 38.9, 20.8 ¹⁹F NMR (376 MHz, Methylene Chloride-*d*₂) δ -152.94. HRMS (ESI) (*m*/*z*): [M]+ calcd. For C₂₁H₁₇N⁷⁹Br: 362.0539, found: 362.0542.

### Example 2: Screening of acridinium-based photoredox catalysts in oxidation of β-O-4 lignin's model to aromatics

To test the performance of the modular acridinium catalysts, 2-(2-methoxyphenoxy)-1-phenylethanol (**1**) was selected as a simple nonphenolic *β*-O-4 -type model substrate owing to this linkage is the most abundant in native Lignin (45-60%). The acridinium-based photoredox catalysts **(OPC1** to **8)** from example 1 were tested. Results are summarized in Table 2 below.

The reaction was carried out in the presence of 0.75 mol% of OPC in DMSO/MeOH/water as solvent mixtures in 8:8:1 mixing ratio under atmospheric pressure of O₂ and blue LEDs irradiation for 12h. We firstly examined the catalytic activity of commercially available Fukuzumi's catalyst, but a moderate conversion of **1** was observed with the formation of oxidized product **2** in only 5% (Table 2, entry 1).

Surprisingly, 1-phenylethane-1,2-diol (**3**) resulting from the C-O bond breakage was the major product, albeit in a very poor yield. This highlights the fact that acridiniums can be an effective OPC for oxidative C-O bond cleavage of the lignin's models using oxygen associated with visible-light as sustainable oxidant systems, all encouraging us to screen other acridinium-based structures to obtain higher yield in favor of **3.** 9-Phenyl-10-methylacridium, bromo, ester, and acetyl, para-substituted 9-phenyl-10-methylacridiums give a similar trend of reactivity (Table 2, entries 2-5).

Interestingly, 10-methylacridium substituted by an *ortho*-substituted aryl group at C9-position give higher conversion. When the reaction is carried out with the photocatalyst **OPC5** with a 2-bromophenyl at the C9 position, the C-O bond cleavage occurred in high yield affording **3** in 84% isolated yield without the formation of overoxidized products **4** and **5** (Table 2, entry 6). The catalyst **OPC6** and **OPC7** with 2-iodophenyl and 2-biphenyl C9-substituent, respectively, gives a slightly lower yield in **3,** suggesting that 2-bromophenyl is the best design for this transformation (Table 2, entries 7 and 8). The **OPC8** bearing a 2-bromo and 4-methyl substituents on the C9aryl also displayed a good reactivity affording **3** in 78% yield (Table 2, entry 9).

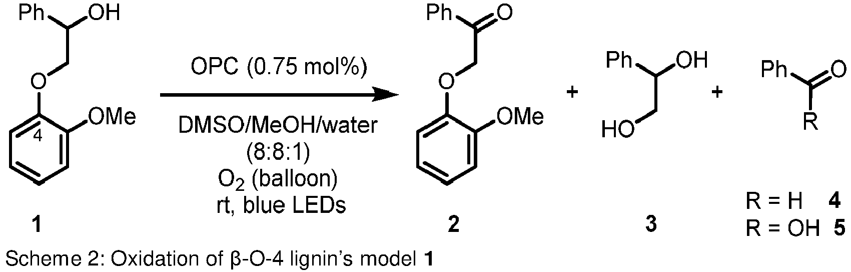

**Table 2: Fukuzumi and OPCs oxidation of β-O-4 lignin's model 1 results**

| Entry | OPC | Conversion (%) | Yield in **2** (%) | Yield in **3** (%) | Yield in **4** (%) | Yield in **5** (%) |
|---|---|---|---|---|---|---|
| 1 | **Fukuzumi** (CAS 1442433-71-7) | 20 | 5 | 10 | trace | 8 |
| 2 | **OPC1** | 35 | 8 | 15 | <5 | 9 |
| 3 | **OPC2** | 28 | 9 | 12 | <5 | <5 |
| 4 | **OPC3** | 30 | <5 | 12 | <5 | 12 |
| 5 | **OPC4** | 31 | <5 | 15 | 7 | 11 |
| 6 | **OPC5** | 100 | 0 | 89 (84) | 0 | 0 |
| 7 | **OPC6** | 61 | - | 52 | 0 | 0 |
| 8 | **OPC7** | 82 | - | 58 | 10 | 12 |
| 9 | **OPC8** | 100 | 21 | 78 | 0 | 0 |
| The conversion is based on **1** consumption, and the yields are determined by **CG**-analysis using biphenyl as the internal standard. Isolated yield is shown in parentheses | | | | | | |

Compounds according to the invention all gave better conversion percentage than the Fukuzumi catalyst. They all represent a better alternative to lignin depolymerisation.

### Example 3: Scope of β-O-4 lignin's model substrates in photocatalytic C-O bond cleavage

### General procedure for the in photocatalytic C-O bond cleavage:

To a 25 mL Schlenk tube was added Lignin model (0.5 mmol), 9-(2-Bromophenyl)-10-methylacridin-10-ium tetrafluoroborate **OPC5** (1.6 mg, 0.00375 mmol, 0.75 mol%,), DMSO (2.3 mL), MeOH (2.3 mL) and water (0.4 mL) were successfully added. After purging the flask three times with vacuum and two times with argon, O₂ atmosphere was incorporated through an O₂-filled balloon. The Schlenk was positioned on a stir plate approximately 2 - 3 cm from two ABI PAR38 (24W) LED lamp supplying blue light (λ = 440- 460 nm). Fans were used for cooling. After 12 hours irradiation, the reaction was quenched by addition of 20 ml CH₂Cl₂ and the crude mixture was washed with 3mL water 3 times, then solvent was removed under reduced pressure and purified by flash column chromatography on silica gel to give the pure product.

A variety of lignin model systems were subjected to the photooxidative catalytic **OPC5** to probe the scope (Scheme 2). Results are summarized in table 3.

Firstly, the methoxy substitution pattern of the phenolic part on the simplified β-O-4 model systems was varied. From 2-(3,5-dimethoxyphenoxy)-1-phenylethanol (**6**) and 2-(3,4-dimethoxyphenoxy)-1-phenylethanol (**7**), the optimized conditions lead to the selective C-O bond cleavage affording the 1-phenylethane-1,2-diol (**2**) in 71% and 75% yield, respectively. These results further prompted the examination of other β-O-4 model systems **8-10,** which are good representations of the different units present in native lignin (coumaryl, coniferyl, and sinapyl alcohols). Using 2-(2-methoxyphenoxy)-1-(4-methoxyphenyl)ethan-1-ol (**8**) in which both arene rings display similar electronic properties the photocatalytic C-O bond cleavage affords the 1-(4-methoxyphenyl)ethane-1,2-diol (**14**) in only 45% yield. However, when the phenolic part contains two methoxy groups such as in **9** or **10,** the diol **14** was isolated in better yields. Again, the C-O bond cleavage of **11,** in which both rings have methoxy substituent, occurred to afford **16** in 46% yield, while **16** is obtained in better yield when the phenol part contains two methoxy group whatever its substitution pattern 3,5 or 3,4 (from **12** and **13**).
**1-Phenylethane-1,2-diol (2):** ¹H NMR (400 MHz, CDCl₃) δ 7.37 - 7.30 (m, 5H), 4.81-4.79 (m, 1H), 3.74-3.71 (m, 1H), 3.64-3.62 (m, 1H), 2.92 (s, 2H). ¹³C NMR (100 MHz, CDCl3) δ 140.4, 128.5, 128.0, 126.1, 74.7, 68.1.
**1-(4-Methoxyphenyl)ethane-1,2-diol (14):** ¹H NMR (400 MHz, CDCl₃) δ 7.23 (2H, d, *J* = 6.6 Hz), 6.86 (2H, d, *J* = 6.6 Hz), 4.71 (1H, d, *J* = 8.0 Hz), 3.78 (3H, s), 3.69 - 3.54 (2H, m), 3.46 (1H, s), 3.11 (1H, s). ¹³C NMR (100 MHz, CDCl3) δ 159.4, 132.7, 127.4, 114.0, 77.4, 77.1, 76.9, 74.4, 68.1, 55.3.
**1-(3-Methoxyphenyl)ethane-1,2-diol (15):** ¹H NMR (400 MHz, CDCl₃) δ = 7.25 (t, *J* = 7.8Hz,1H), 6.92 (d, *J* = 9.0Hz,2H), 6.83 (d, *J* =8.4Hz, 1H), 4.77 (dd, *J* = 8.4, 4.8 Hz, 1H), 3.79 (s, 3H), 3.74-3.71 (m, 1H), 3.65-3.61 (m, 1H), 2.05 (br s, 2H). ¹³C NMR (100 MHz, CDCl₃) δ 159.9, 142.4, 129.7, 118.5, 113.5, 111.8, 74.7, 68.2, 55.4.

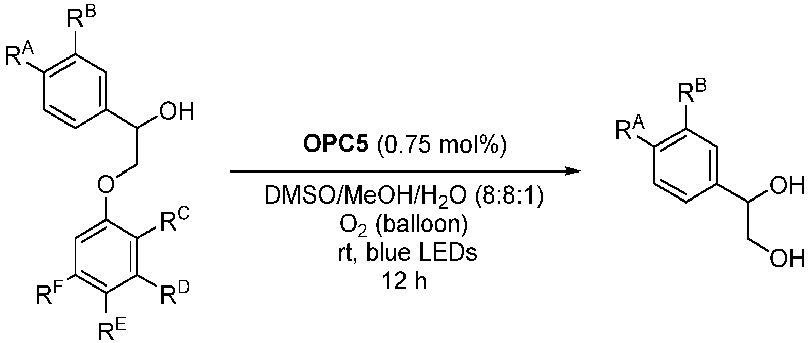

**Table 3: Scope of β-O-4 Lignin's Model Substrates in Photocatalytic C-O Bond Cleavage**

| | β-O-4 Lignin's Model Substrates | | | | | | | Diols | |
|---|---|---|---|---|---|---|---|---|---|
| Entry | Name | R^{A} | R^{B} | R^{C} | R^{D} | R^{E} | R^{F} | Name | Yield (%) |
| 1 | **6** | H | H | H | OMe | H | OMe | **3** | 71 |
| 2 | **7** | H | H | H | OMe | OMe | H | **3** | 75 |
| 3 | **8** | OMe | H | OMe | H | H | H | **14** | 45 |
| 4 | **9** | OMe | H | H | OMe | OMe | H | **14** | 78 |
| 5 | **10** | OMe | H | H | OMe | H | OMe | **14** | 90 |
| 6 | **11** | H | OMe | OMe | H | H | H | **15** | 46 |
| 7 | **12** | H | OMe | H | OMe | OMe | H | **15** | 88 |
| 8 | **13** | H | OMe | H | OMe | H | OMe | **15** | 86 |

In conclusion, a novel approach toward the valorisation of lignin and related systems has been developed by designing a novel organic photoredox catalyst. Various lignin model compounds underwent a smooth transformation to afford the corresponding C-O bond cleavage product in moderate to excellent yields under mild reaction conditions using oxygen and visible-light as oxidant. The unique photoredox properties of this acridinium-based catalysts according to the invention have allowed the selective formation of 1-arylethane-1,2-diols in high yields that constitutes a vital step toward converting lignin to value-added, low-molecular-weight aromatics.

### List of references

**[1]** J. Zakzeski, P. C. A. Bruijnincx, A. L. Jongerius, B. M. Weckhuysen, Chem. Rev. 2010, 110, 3552-3599.
**[2]** a) C. Li, X. Zhao, A. Wang, G. W. Huber, T. Zhang, Chem. Rev. 2015, 115, 11559-11624; b) M. D. Kärkäs, B. S. Matsuura, T. M. Monos, G. Magallanes, C. R. J. Stephenson, Org. Biomol. Chem. 2016, 14, 1853-1914.
**[3]** Y.-T. Cheng, J. Jae, J. Shi, W. Fan, G. W. Huber, Angew. Chem. Int. Ed. 2012, 51, 1387-1390.
**[4]** A. Rahimi, A. Ulbrich, J. J. Coon, S. S. Stahl, Nature 2014, 515, 249-252.
**[5]** J. Reiter, H. Strittmatter, L. O. Wiemann, D. Schieder, V. Sieber, Green Chem. 2013, 15, 1373-1381.
**[6]** J. Zhang, J. Teo, X. Chen, H. Asakura, T. Tanaka, K. Teramura, N. Yan, ACS Catal. 2014, 4, 1574-1583.
**[7]** A. Rahimi, A. Azarpira, H. Kim, J. Ralph, S. S. Stahl, J. Am. Chem. Soc. 2013, 135, 6415-6418.
**[8]** a) M. D. Kärkäs, I. Bosque, B. S. Matsuura, C. R. J. Stephenson, Org. Lett. 2016, 18, 5166-5169; b) I. Bosque, G. Magallanes, M. Rigoulet, M. D. Kärkäs, C. R. J. Stephenson, ACS Cent. Sci. 2017, 3, 621-628; c) N. Luo, M. Wang, H. Li, J. Zhang, H. Liu, F. Wang, ACS Catal. 2016, 6, 7716-7721; d) J. Luo, J. Zhang, J. Org. Chem. 2016, 81, 9131-9137; e) J. Luo, X. Zhang, J. Lu, J. Zhang, ACS Catal. 2017, 7, 5062-5070.
**[9]** J. Xie, J. Qi, C.-Y. Hse, T. F. Shupe, BioResources 2014, 9, 578-588.
**[10]** C. Zhu, W. Ding, T. Shen, C. Tang, C. Sun, S. Xu, Y. Chen, J. Wu, H. Ying, ChemSusChem 2015, 8, 1768-1778.
**[11]** K. Ohkubo, K. Suga, S. Fukuzumi, Chem. Commun. 2006, 2018-2020.
**[12]** A. Gini, M. Uygur, T. Rigotti, J. Alemán, O. Garcia Mancheño, Chem. Eur. J. 2018, 24, 12509-12514.

## Claims

1. A compound of formula I : 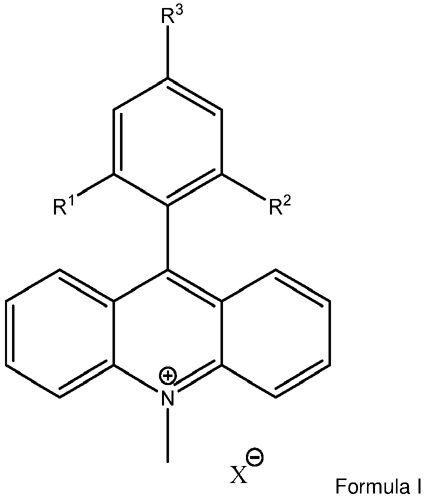 wherein
- the R¹ and R² groups independently represents H, a halo group or a phenyl group,
- the R³ group represents H, a halo group, a C1 to C4 linear or branched alkyl chain, a -COOR group, a -COR group or a phenyl group, in which R represents a C1 to C4 linear or branched alkyl chain, and
- X⁻ represents an anion.

2. The compound according to claim 1, wherein at least one group among R¹, R² or R3 is not H.

3. A compound of formula II: 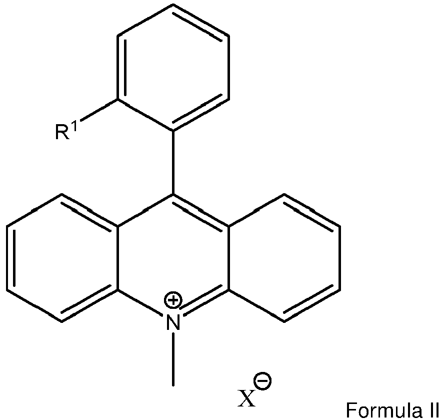 wherein
- the R¹ group represents a halo group or a phenyl group and
- X⁻ represents an anion.

4. A compound of formula III: 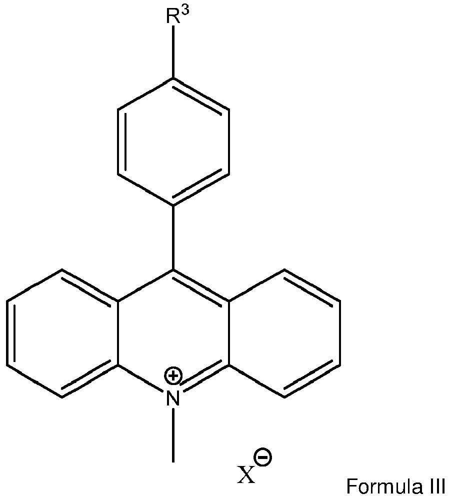 wherein,
- the R³ group represents a halo group, a C1 to C4 linear or branched alkyl chain, a - COOR group a -COR group or a phenyl group, in which R is a C1 to C4 linear or branched alkyl chain, and
- X⁻ represents an anion.

5. The compound according to any of preceding claims, wherein the anion X⁻ is chosen in the group comprising BF₄⁻, PF₆⁻, Cl⁻, ClO₄⁻ preferably BF₄⁻.

6. Use of the compound according to any of preceding claims as a catalyst, preferably in a C-O bond cleavage, and more preferably a photocatalytic C-O bond cleavage.

7. Use of the compound according to any of preceding claims in a reaction of lignin depolymerisation, preferably in oxidative depolymerisation of lignin.

8. A synthetic method of a compound of formula I comprising a step of photoreacting, in an organic solvent, a 10-methylacridinium salt and a diazonium salt of formula I': 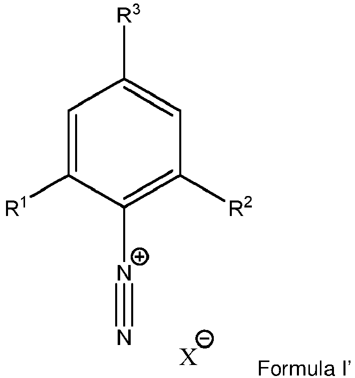 wherein,
- the R¹ and R² groups independently represent H, a halo group or a phenyl group,
- the R³ group represents H, a halo group, a C1 to C4 linear or branched alkyl chain, a -COOR group, a -COR group or a phenyl group, in which R represents a C1 to C4 linear or branched alkyl chain, and
- X⁻ represents an anion.

9. The method according to preceding claim, wherein at least one group among R¹, R² or R³ is not H.

10. The method according to any of claims 8 to 9, wherein the anion X⁻ is chosen in the group comprising BF₄⁻, PF₆⁻, Cl⁻ and ClO₄⁻, preferably BF₄⁻.

11. The method according to any of claims 8 to 10, wherein the compounds of formula I' are chosen in the group of compounds of formula II': 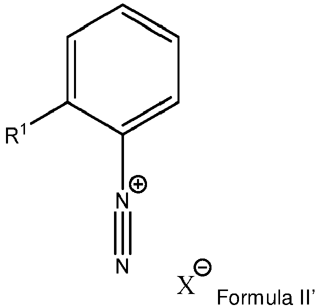 wherein
- R¹ represents a halo group or a phenyl group, and
- X⁻ represents an anion.

12. The method according to any of claims 8 to 10, wherein the compounds of formula I' are chosen in the group of compounds of formula III': 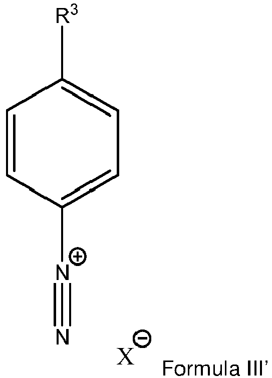 wherein,
- the R³ group represents a halogen, a C1 to C4 linear or branched alkyl chain, a - COOR group, a -COR group or a phenyl group, in which R is a C1 to C4 linear or branched alkyl chain and
- X⁻ represents an anion.

13. The method according to any of claims 8 to 12, wherein the organic solvent is chosen in the group comprising methanol, dimethylsulfoxide, acetonitrile, dichloromethane and mixtures thereof.

14. The method according to any of claims 8 to 13, wherein the 10-methylacridinium salt is chosen on the group comprising BF₄⁻, PF₆⁻, Cl⁻ and ClO₄⁻, preferably BF₄⁻.

15. The method according to any of claims 8 to 14, wherein the photoreaction is done by means of irradiation at a wavelength comprised in the range of visible light, preferably from 400 to 800 nm, and more preferably from 440 to 460 nm.

16. The method according to any of claims 8 to 15, wherein the reaction is done at a temperature comprised in a range from 10 to 45 °C, preferably 30 ºC.
